# EUROPÄISCHE PATENTSCHRIFT

(11) **EP 2 814 406 B1**
(45) Veröffentlichungstag und Bekanntmachung des Hinweises auf die Patenterteilung: **01.04.2020**
(21) Anmeldenummer: 13713724.6
(22) Anmeldetag: 07.02.2013
(51) Int. Cl.: A61B 17/122, A61B 17/128

(54) **VORRICHTUNG ZUM APPLIZIEREN EINES CHIRURGISCHEN CLIPS**
DEVICE FOR APPLYING A SURGICAL CLIP
DISPOSITIF PERMETTANT L'APPLICATION D'UNE PINCE CHIRURGICALE

(30) Priorität: 16.02.2012 DE 102012003334
(43) Veröffentlichungstag der Anmeldung: 24.12.2014
(73) Patentinhaber: Otten, Brigitte, 27610 Schiffdorf (DE); Honnefelder, Anja, 20249 Hamburg (DE); Otten, Peer, 20146 Hamburg (DE)
(72) Erfinder: OTTEN, Gert, 27610 Schiffdorf (DE); OTTEN, Brigitte, 27610 Schiffdorf (DE)
(74) Vertreter: Müller, Wolfram Hubertus
(86) Internationale Anmeldenummer: PCT/DE2013/000073
(87) Internationale Veröffentlichungsnummer: WO 2013/120477

(56) Entgegenhaltungen:
- EP-B1- 1 611 853
- US-A- 3 510 923

## Beschreibung

Die Erfindung betrifft eine Anordung mit einem chirurgischen Clip, wie er zunehmend bei endoskopischen Eingriffen in der minimal invasiven Chirurgie zum Abklemmen und Unterbrechen von Weichteilgewebe und Blutgefäßen benötigt wird, und mit einer Kartusche zum Applizieren des chirurgischen Clips.

Bekannte chirurgische Clips haben überwiegend eine U- oder V-förmige Form mit zwei sich gegenüberstehenden Klemmbalken mit speziellen Formkonturen. Sie werden mit dafür geeigneten Handhabungsinstrumenten, sogenannten Applikatoren, unter Kraftschluss in die gewünschte Greif- und Schließstellung gebracht. Clips, die aus Titan gefertigt sind, verbleiben dann im Körper. Sie können auch aus einem resorbierbarem Kunststoff, beispielsweise einem Polypeptid, bestehen, der im Körper enzymatisch abgebaut wird.

Einen Clip zum Verschließen von Blutgefäßen beschreibt DE 20 2005 009 061 U1. Ein Grundkörper wird mit seiner Innenseite an das geöffnete Blutgefäß gelegt und mit mehreren am Grundkörper biegbar angebrachten abstehenden Beinen durch Umbiegen dieser Beine verschlossen. Neben einer komplizierten Handhabung bietet dieser Clip keine ausreichende Sicherheit gegen die Verletzung von Gewebe.

Die DE 10 2008 031 387 A1 offenbart einen medizinischen Clip zum Verschließen einer Gewebeöffnung. Zwei axial auf einer Drehachse gelagerte Greifarme, deren Ende jeweils zueinander abgewinkelt ist, erhalten im Fall des gegenseitigen Eingriffs durch eine Federkraft oder durch einen Rastmechanismus die zum Verschließen der Gewebeöffnung erforderliche Greifkraft. Das Gewebe wird im Wesentlichen gerafft. Ein gezieltes Clippen ist mit dieser technischen Lösung nur eingeschränkt möglich. Ebenso ist der Anwendungsbereich begrenzt und sind zum Beispiel Blutgefäße ausgenommen.

Zum Abklemmen und Unterbrechen von Blutgefäßen geht aus DE 10 2006 031 092 B3 ein chirurgischer Clip hervor, der aus zwei über ein Federelement miteinander verbundenen und sich überkreuzenden Armen, die an ihrem Ende jeweils Klemmbacken tragen, besteht. Um eine reproduzierbare und nicht verschiebbare Anlage des Clips zu ermöglichen, weisen die Arme quer zu ihrer Längsrichtung beidseitig Öffnungen auf, in die stift- oder zapfenförmige Fixierelemente greifen können.

Einen als Implantat vorgesehenen Clip offenbart DE 20 2010 008 512 U1. Zwei Clipbranchen, die an ihrem ersten freien Ende durch ein federelastisches Element parallel aneinander anliegend gehalten werden, und ein zweites Ende aufweisen, wobei die beiden Enden jeweils durch sich überkreuzend ausgebildete Abschnitte verbunden sind. Zum Anlegen des Clips in eine Applikationsstellung werden die zweiten Enden durch eine Clipanlegezange gefasst und gegen die Federkraft des federelastischen Elementes angenähert. Um eine präzise Schließkraft zu erreichen, weisen die Clipbranchen komplementär ausgebildete Anschlagelemente auf. Diese Lösung ist für die minimal invasive Chirurgie nicht geeignet. Durch die vorhandene Scherwirkung besteht außerdem die Gefahr, dass die Branchen bei deren Anwendung auseinandergedrückt werden.

Einen weiteren chirurgischen Clip beschreibt DE 20 2010 008 714 U1. Er besteht aus zwei Clipbranchen, deren ersten freien Enden in Ruhestellung durch die vorgegebene Schließkraft eines federelastischen Elementes parallel zueinander gehalten werden, während die zweiten ebenfalls freien Enden am federelastischen Element angebracht sind. Um den Clip anzulegen, werden die zweiten freien Enden mit einem speziell dafür vorgesehenen Greifwerkzeug gefasst und miteinander in Kontakt gebracht, so dass sich die Clipbranchen öffnen und diese angelegt werden können. Durch Entlastung der zweiten freien Enden ist der Clip positioniert. Die Herstellung dieses Clips erscheint kompliziert und besonders aufwendig zu sein.

Zum Abklemmen von organischem Gewebe, insbesondere von Blutgefäßen, beschreibt DE 10 2004 015 224 A1 eine chirurgische Klammer aus resorbierbarem Material, die aus zwei V-förmig angeordneten Schenkeln gebildet ist und ein Schenkel an seinem Ende einen Haltestift aufweist, der zum Abklemmen des organischen Gewebes in eine am Ende des anderen Schenkels vorgesehene Ausnehmung greift und dort fixiert ist. Die Anwendung für axiale Clippungen schränkt die Verwendung dieses Clips ein.

Für die Anwendung in der offenen Chirurgie ist die aus DE 10 2004 015 223 A1 hervorgehende chirurgische Klammer geeignet. Zwischen zwei parallel zueinander angeordneten Klemmbalken kann das zu verbindende organische Gewebe positioniert werden, das danach mittels an einem Klemmbalken quer zu dessen Längsachse angeordneten und in passfähige Bohrungen des anderen Klemmbalkens greifende Stifte fixiert werden kann.

US 4,7651,737 offenbart eine Vorrichtung zum Abklemmen von organischem Gewebe, insbesondere von Blutgefäßen, unter Verwendung eines Satzes Klemmstäbe. Ein am distalen Ende angeordneter Klemmstab ist mittels einer Zapfverbindung lösbar mit einem Applikationssteg verbunden. Auf diesem kann der andere Klemmstab in horizontaler Lage unter Abklemmen des organischen Gewebes gegen den am distalen Ende angeordneten Klemmstab geschoben und zusammen mit diesem und dem abgeklemmten organischen Gewebe vom Applikationssteg gelöst werden.
Mit dieser technischen Lösung verbindet sich jedoch das Problem, dass die Klemmstäbe mit ihren zum Teil verzahnten Klemmstegen einen vorgegebenen und nicht veränderbaren Abstand beim Schließen zueinander aufweisen, da sie nicht gegeneinander, sondern in der Endphase übereinander geschoben werden und so bei dem organischen Gewebe eine Scher-Klemmwirkung mit der großen Gefahr des Verletzens von Gewebe bis hin zum ungewollten Durchtrennen von Gewebe erzeugen. Ein stufenloses vom Kompressionsdruck abhängiges Abklemmen von organischem Gewebe ist dadurch objektiv nicht möglich. Die in US 4,7651,737 beschriebene technische Lösung lässt insofern kein stufenloses Zusammenführen der Klemmstäbe zum Abklemmen des Gewebes zu. Das Abklemmen erfolgt, ohne die Art des Gewebes und die vorhandenen sonstigen Bedingungen berücksichtigen zu können. Der sich durch das Übereinanderschieben der Klemmstäbe bildende Spalt hat eine unveränderbare Größe, so dass die Gefahr besteht, dass Gewebe, insbesondere kleine Blutgefäße, nur unzureichend abgeklemmt werden und Blutungen bei einer Operation die Folge sein können.

Mit dem Ziel, die genannten Probleme zu überwinden, schlägt EP 1 611 853 B1 eine chirurgische Vorrichtung zum Abklemmen organischen Gewebes, insbesondere von Blutgefäßen, unter Verwendung eines auf einem Applikationssteg angeordneten Satz von Klemmbalken vor.
Die Klemmbalken weisen an ihrer Basis zur Positionierung und zum horizontalen Verschieben in der Vorrichtung jeweils beidseitig mindestens einen Führungsstift auf. Zum Erzeugen einer Gewebeklammer besitzt der am distalen Ende angeordnete Klemmbalken an seiner Basis eine Haltebohrung, die mit dem an der Basis des anderen Klemmbalkens vorgesehenen Haltestift korrespondiert und nach dem Zusammenschieben der Klemmbalken mittels eines Stößels unter Abklemmen des Gewebes im Applikator die Gewebeklammer bildet. Die Führungsstifte sind dabei so dimensioniert, dass sie bei einem Überschreiten des für das Abklemmen des Gewebes erforderlichen Kraftaufwandes abscheren und die gebildete Gewebeklammer dann leicht mittels des zentral gelagerten Stößels vom Applikationssteg geschoben werden kann.

Die Anwendung dieser technischen Lösung zeigt eine einfache Handhabung des Applikators und ermöglicht eine sichere Fixierung des abgeklemmten Gewebes in der Klammer. Es hat sich jedoch auch gezeigt, dass die so erzeugte Gewebeklammer in ihrer Längsrichtung noch nicht den gewachsenen Anforderungen genügt, insbesondere hinsichtlich für eine hohe Stabilität der Gewebeklammer erforderlichen Kompressionsdruckes zwischen den Klemmbalken
Das zu klemmende Gewebe ist in seiner Struktur sehr inhomogen, so weisen Blutgefäße einen höheren Klemmwiderstand als lockeres Bindegewebe auf. In Abhängigkeit vom punktuellen Gewebswiderstand und dem ausgeübten Klemmdruck ist nicht auszuschließen, dass die Klemmbalken infolge unzureichender Stabilität des Haltestiftes der Klammer V-förmig auseinanderweichen und so zu einem teilweisen oder vollständigen Verlust der Funktion des Clips führen können.

Aufgabe der Erfindung ist es daher, eine Anordnung mit einem verbesserten chirugischen Clip und mit einer Kartusche zum Applizieren des chirurgischen Clips bereitzustellen, wobei der Clip bei Beibehaltung einer gewebeschonenden Anwendung ein stabiles und torsionssteifes Applizieren ermöglicht und eine dauerhafte und weitgehend homogene Gewebekompression zwischen den Klemmbalken ermöglicht sowie eine hohe Verschlusssicherheit der Gewebeklammer sowohl hinsichtlich einer rotationsstabilen Position als auch in der Längsrichtung der Gewebeklammer sichert.

Erfindungsgemäß wird die Aufgabe durch eine Anordnung mit einem chirurgischen Clip für die minimal invasive Chirurgie zum Abklemmen von organischem Gewebe zu einer Gewebeklammer und mit einer Kartusche zum Applizieren des chirurgischen Clips mit den Merkmalen des Patentanspruchs 1 gelöst. Ausgestaltungen der Erfindung sind in den abhängigen Ansprüchen angegeben.

Mit der Erfindung verbindet sich der besondere Vorzug, dass bei noch geöffnetem Clip der am distalen Ende der Kartusche unter Pressklemmung in deren Führungsnut gelagerte erste Klemmbalken vorübergehend als Haken zur Darstellung der zu clippenden Gewebestruktur genutzt werden kann. Durch die sichere Hakenfunktion dieses ersten Klemmbalkens wird es überhaupt erst möglich, bei noch geöffneten Clip die ideale Position für die Clippung im Gewebe zu suchen und festzulegen.

Der zweite Klemmbalken kann in einer Ebene stufenlos und in jeder Situation kontrollierbar mit dem ersten Klemmbalken zusammengeführt werden, wobei das Gewebe genau an der gewünschten Stelle durch die stufenfreie Pressklemmung ausreichend schonend gequetscht jedoch nicht zerquetscht und damit eine Perfusion im organischen Gewebe unterbrochen wird.

Diese neue Möglichkeit des Clipsetzens schließt eine fehlerhafte Platzierung des Clips und damit ernste Folgen für den Patienten aus. Die Gewebeklammer mit ihrer hohen Verschlusssicherheit ist eine für die minimal invasive Chirurgie besonders geeignete Gewebeklammer, da das Setzen der Clippung in jeder Phase unter uneingeschränkter Sicht auf das OP-Feld erfolgt und damit eine fehlerhafte Platzierung des Clips ausgeschlossen wird.

Haltestift und Haltebohrung sind in ihrer nicht kreisrunden Formgebung und in ihrem Querschnitt korrespondierend zueinander ausgeführt. Der erfindungsgemäße Clip ermöglicht, dass das Gewebe ausschließlich durch einen stufenlosen Pressklemm- Mechanismus abgeklemmt wird und die beiden Klemmbalken mit dem abgeklemmten Gewebe sowohl in einer torsionssteifen als auch in Längsrichtung der Gewebeklammer dauerhaft stabilen Position gehalten werden.

Durch den zusätzlichen Schenkel, der sich formschlüssig bei Abklemmen des organischen Gewebes unter den kürzeren korrespondierenden Klemmbalken schiebt, wird der Haltestift besonders von Biegebeanspruchung entlastet, die notwendig bleibende Parallelität der Klemmbalken in jeder Phase des Clipvorganges gewährleistet und damit der Gewebeklammer die erforderlich hohe Stabilität gegeben.

Die Stabilität der Gewebeklammer ist somit sowohl durch den formschlüssig unter den kürzeren korrespondierenden Klemmbalken platzierten Schenkel des längeren Klemmbalkens als auch durch die Klemmpressung des von einer Biegebeanspruchung entlasteten Haltestiftes gewährleistet. Ein Aufbiegen der beiden Klemmbalken beim Schließen des Clips und in dessen geschlossenem Zustand wird dadurch vermieden. Der Clip behält seine Stabilität bis zur abgeschlossenen Wundheilung.
Die Erfindung ermöglicht darüber hinaus eine flexible Adaption an unterschiedliche Dicken des Gewebes.
Der erfindungsgemäße Clip kann in einer extrem filigranen Ausführung gefertigt werden und besitzt daher eine für die minimal invasive Chirurgie sehr bedeutsame extreme Raumbeweglichkeit.
Die Erfindung erfüllt damit ein in der minimal invasiven Chirurgie bestehendes dringendes Bedürfnis nach einem Höchstmaß an Sicherheit beim Setzen des Clips und gewährleistet zugleich höchste Sicherheit für Patienten und Arzt. Mit der Erfindung verbindet sich daher ein wesentlicher technischer Fortschritt.

Bei einer besonderen Ausführungsform der Erfindung sind die Schultern des ersten Klemmbalkens zum distalen Ende hin über ihre gesamte Länge oder nur teilweise abgeflacht ausgebildet, jeweils korrespondierend mit einer Verringerung der lichten Weite zwischen den Nuten der Kartusche.

Nach einer weiteren Ausführungsform sind die Klemmflächen des ersten und des zweiten Klemmbalkens aufgeraut oder wellenförmig ausgebildet.

Eine besonders bevorzugte Ausführungsform sieht vor, dass die Klemmfläche des ersten Klemmbalkens und die Klemmfläche des korrespondierenden zweiten Klemmbalkens stufenförmig ausgebildet sind.

Gemäß einer weiteren Ausführungsform der Erfindung weist der Schenkel eine Länge aufweist, die mindestens der Längsbreite des korrespondierenden ersten Klemmbalkens an dessen Basis entspricht.

Eine weitere Ausführungsform der Erfindung sieht vor, dass die Schultern des ersten Klemmbalkens vertikal verlaufende Rippungen aufweisen.

Nach einer besonders bevorzugten Ausführungsform des erfindungsgemäßen Clips wird die Klemmpressung des ersten Klemmbalkens durch die in Richtung des distalen Endes der Kartusche verlaufende Verringerung der lichten Weite zwischen den Führungsnuten und/oder durch die Rippen der Schultern des ersten Klemmbalkens bewirkt, wobei die Rippen in die Ausnehmungen greifen, die in den Führungsnuten am distalen Ende der Kartusche dafür vorgesehen sind.

Vorteilhafterweise entspricht die Breite des Schenkels der Breite des Bettes der Kartusche.

Nach einer weiteren Ausführungsform der Erfindung weisen die Schultern des ersten Klemmbalkens und es zweiten Klemmbalkens eine solche Elastizität auf, dass die Gewebeklammer auch bei starren distalen Enden der Kartusche durch Pressdruck unbeschadet auswerfbar ist.

Gemäß einer weiteren Ausführungsform der Erfindung bestehen die Schultern des ersten Klemmbalkens und des zweiten Klemmbalkens aus einem unelastischen Material und ist das distale Ende der Kartusche elastisch ausgebildet, wodurch ein unbeschadeter Auswurf der Gewebeklammer ermöglicht ist.

Nach einer besonders bevorzugten Ausführungsform ist das Mittel zur lösbaren Verbindung mit dem Applikator ein Anschlag und ein am unteren Ende eines Hüllrohrabschnittes der Kartusche vorgesehenes Fixierelement für die Aufnahme des Applikators mittels eines Bajonettverschlusses oder das Mittel sind Rastelemente.

Nach einer besonderen Ausführungsform der Erfindung weist der Stößel ein Fixierelement auf, das bei Handhabung des chirurgischen Clips in korrespondierende Ausnehmungen greift, die mit Abstand voneinander in Längsrichtung im Boden des Hüllrohrabschnittes vorgesehen sind.

Im Folgenden soll die Erfindung an Hand von Zeichnungen, die den chirurgischen Clip in wesentlich vergrößerter und prinzipienhafter Darstellung zeigen, näher erläutert werden. Es zeigen
- **Fig. 1**: die Seitenansicht des Clips in Prinzip-Darstellung;
- **Fig. 1a**: die Draufsicht auf den Klemmbalken der **Fig. 1** mit Haltestift;
- **Fig. 1b**: die Draufsicht auf den Klemmbalken der **Fig. 1** mit Haltebohrung;
- **Fig. 2**: die Seitenansicht einer speziellen Ausführungsform des Clips in Prinzipdarstellung;
- **Fig. 2a**: die Draufsicht auf den Klemmbalken der **Fig. 2** mit Haltestift;
- **Fig. 2b**: die Draufsicht auf den Klemmbalken der **Fig. 2** mit Haltebohrung;
- **Fig. 3**: die Seitenansicht einer weiteren speziellen Ausführungsform des Clips in Prinzip-Darstellung;
- **Fig. 3a**: die Draufsicht auf den Klemmbalken der **Fig. 3** mit Haltestift;
- **Fig. 3b**: die Draufsicht auf den Klemmbalken der **Fig. 3** mit Haltebohrung;
- **Fig. 4**: die Seitenansicht einer weiteren speziellen Ausführungsform des Clips in Prinzip-Darstellung;
- **Fig. 4a**: die Draufsicht auf den Klemmbalken der **Fig.** 4 mit Haltestift;
- Fig. 4b: die Draufsicht auf den Klemmbalken der **Fig. 4** mit Haltebohrung;
- **Fig. 5**: die Seitenansicht einer weiteren Ausführungsform des Clips;
- **Fig. 6**: die Seitenansicht einer weiteren Ausführungsform des Clips;
- **Fig. 6a**: die Draufsicht auf den Klemmbalken der **Fig. 6** mit Haltestift;
- **Fig. 6b**: die Draufsicht auf den Klemmbalken der **Fig. 6** mit Haltebohrung;
- **Fig. 7**: teilweise im Längsschnitt die prinzipielle Darstellung eines Klemmbalkens in einer Kartusche;
- **Fig. 8**: die Seitenansicht einer Kartusche mit eingesetztem Clip in Prinzip-Darstellung;
- **Fig. 9**: die Vorderansicht der Kartusche gemäß **Fig. 8****;**
- **Fig. 10a**: die Seitenansicht eines Stößels;
- **Fig. 10b**: die Vorderansicht des Stößels gemäß **Fig. 10a****.**

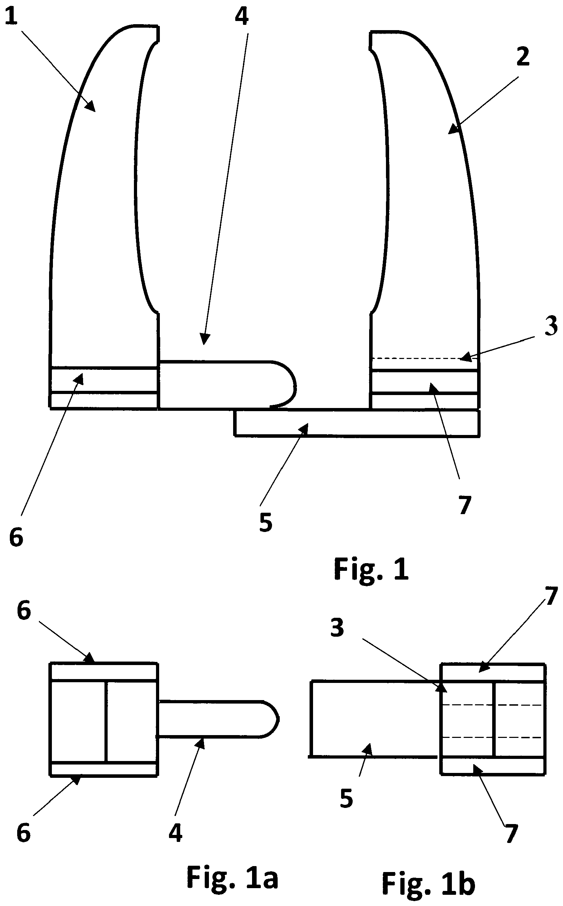

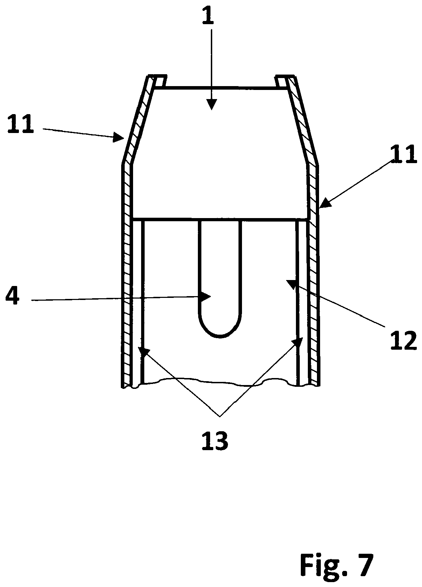

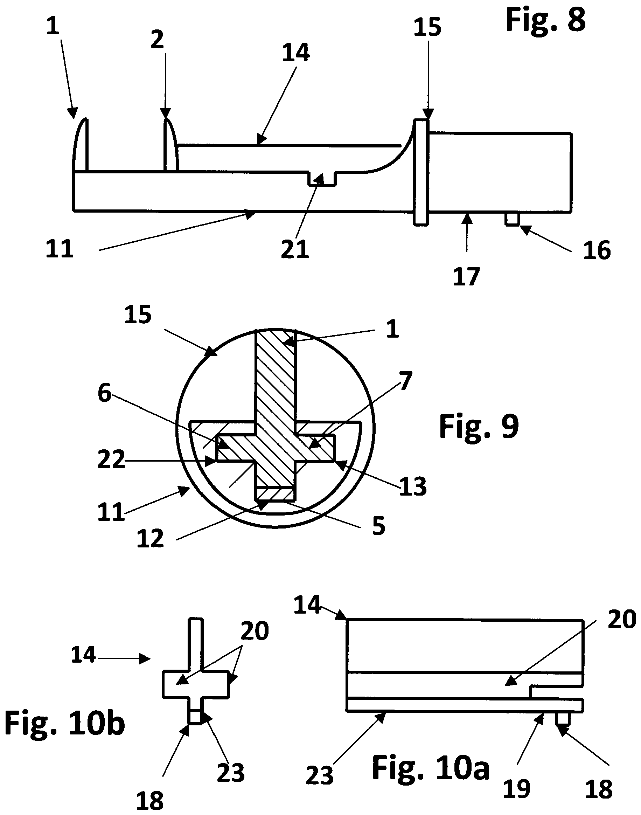

Wie aus **Fig. 1** sowie den **Figuren 1a** und **1b** zu ersehen ist, besteht der erfindungsgemäße Clip aus den miteinander korrespondierenden ersten Klemmbalken 1 und zweiten Klemmbalken 2, deren Klemmflächen konkav ausgebildet sind und erforderlichenfalls auch eine aufgeraute, wellenförmige oder konvexe Oberfläche aufweisen können. Die Klemmbalken 1, 2 können auch plan ausgebildete Klemmflächen besitzen. An der Basis der Klemmbalken 1, 2, die aus einem bekannten resorbierbaren Material bestehen, sind die Schultern 6 und 7 vorgesehen, mit denen die Klemmbalken 1, 2 in passfähigen Nuten 13, 22 (**Fig. 9**) der zu ihrem Applizieren zu verwendenden Kartusche 11 horizontal geführt werden, nachdem die Klemmbalken 1, 2 zuvor durch passfähige Einsatzöffnungen 21 (**Fig. 8**) in die Kartusche 11 eingeführt und in Position gebracht wurden.
Erfindungswesentlich ist der an der Basis des zweiten Klemmbalkens 2 angeordnete Schenkel 5, der vorzugsweise die Breite des Bettes 12 der Kartusche 11 (**Fig. 9**) ausfüllt. Der erste Klemmbalken 1 mit dem Haltestift 4 ist am distalen Ende in der hier nicht dargestellten Kartusche 11 mittels Pressklemmung positioniert. Zum Clippen von Gewebe wird der zweite Klemmbalken 2 zum distalen Ende hin geschoben, wobei die Haltebohrung 3 des zweiten Klemmbalkens 2 um den Haltestift 4 des ersten Klemmbalkens 1 greift und sich der Schenkel 5 bündig unter den kürzeren ersten Klemmbalken 1 schiebt. Wenn der zweite Klemmbalken 2 zum Clippen von Gewebe gegen den ersten Klemmbalken 1 geschoben wird, gleitet der Schenkel 5 auf dem Bett 12 (**Fig. 7** **und** **Fig. 9**) der hier nicht dargestellten Kartusche 11. Der Schenkel 5 ermöglicht einen dreh- und kippfreien Vorschub des zweiten Klemmbalkens 2 und gewährleistet so eine höchst stabile Präzision beim Zusammenführen des ersten Klemmbalken 1 und des zweiten Klemmbalkens 2 bis zum Ausbilden der Gewebeklammer. Der Schenkel 5 besitzt vorteilhafterweise eine Länge, die mindestens der Längsbreite des jeweils korrespondierenden Klemmbalkens 1, 2 entspricht. Die Haltebohrung 3 weist in ihrem Querschnitt zur Sicherung einer rotationsstabilen Lage der Gewebeklammer eine von einem Kreis abweichende Formgebung auf.
Sobald der durch den hier nicht dargestellten Stößel 14 über den zweiten Klemmbalken 2 auf das erfasste Gewebe und den Klemmbalken 1 ausgeübte Druck die Klemmpressung der Kartusche 11 auf die Schultern 6, 7 des ersten Klemmbalkens 1 übersteigt, wird die durch Kraft- und Formschluss des Haltestiftes 4 in der Haltebohrung 3 gebildete Gewebeklammer mittels des Stößels 14 aus der Kartusche 11 freigegeben. Von Bedeutung ist dabei, dass für die Schultern 6, 7 der Klemmbalken 1, 2 ein resorbierbares Material zum Einsatz kommt, dessen Elastizität auch bei starr ausgebildeten Enden der Kartusche 11 einen unbeschadeten Auswurf der ausgebildeten Gewebeklammer aus deren distalem Ende ermöglicht. Bei Verwendung eines unelastischen Materials für die Schultern 6, 7 der Klemmbalken 1 und 2 ermöglicht die elastische Ausbildung der distalen Enden der Kartusche 11 einen unbeschadeten Auswurf der Gewebeklammer. Die Elastizität des resorbierbaren Materials und die Elastizität der distalen Enden der Kartusche können auch aufeinander abgestimmt sein, um einen unbeschadeten Auswurf der Gewebeklammer zu gewährleisten.
Dem Schenkel 5 kommt in medizinischer, insbesondere chirurgischer Hinsicht, eine besondere Bedeutung zu. Er verleiht der fertigen Gewebeklammer neben der Unterstützung der rotationsstabilen Lage vor allem eine zusätzliche mechanische Stabilität und hohe Sicherheit in der Längsrichtung der Gewebeklammer bei gleichzeitig sicherer Fixierung der Gewebestruktur. Diese zusätzliche Stabilität ist entscheidend von Bedeutung, wenn Clips mit einer größeren Länge der Klemmbalken zum Einsatz kommen. In der Regel kommen Clips mit einer Länge der Klemmbalken 1, 2 zum Einsatz kommen. In der Regel finden Clips mit einer Länge der Klemmbalken von 5 mm bis 20/25 mm Verwendung.
Die Gewebeklammer verfügt mit dem unter dem ersten Klemmbalken 1 positionierten Schenkel 5 und der form- und kraftschlüssigen Verbindung des Haltestiftes 4 in der Haltebohrung 3 über eine stabile zwei-Punkt-Lagerung, die durchgehend einen gleichen Klemmdruck auf das Gewebe zwischen dem ersten Klemmbalken 1 und dem zweiten 2 ausübt.

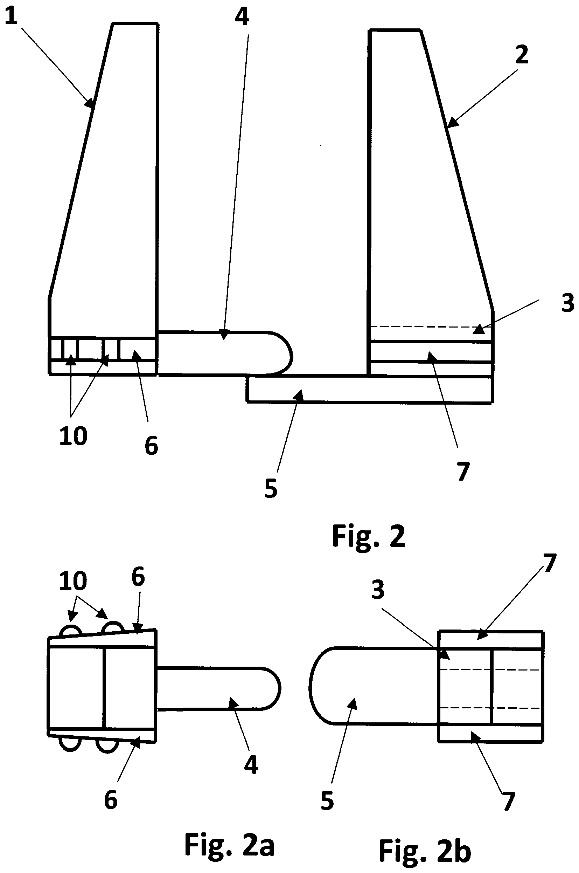

Die **Figuren 2, 2a** und **2b** zeigen in einer Seitenansicht und in Draufsichten eine spezielle Ausführungsform der Erfindung. Wie aus **Fig. 2a** zu entnehmen ist, sind die Schultern 6 des ersten Klemmbalkens 1 zu dessen distalem Ende hin abgeflacht und weisen hier auf jeder Seite jeweils zwei vertikal verlaufende Rippen 10 auf. Der Klemmbalken 1 kann jedoch auch Schultern 6 mit nur einer Rippe 10 oder mehr als zwei Rippen 10 auf jeder Seite versehen werden. Die Kartusche 11 besitzt korrespondierend zu den Rippen 10 an ihren beiden Innenflächen entsprechende Ausnehmungen. Es ist aber auch möglich, dass die Schultern 6, 7 Ausnehmungen für die Rippen aufweisen, die korrespondierend dazu in den Führungsnuten ausgebildet sind. Die Rippen 10 fixieren den ersten Klemmbalken 1 für sich oder zusätzlich zu der durch die Kartusche 11 ausgeübten Pressklemmung. Der stabile und kippsichere Sitz des ersten Klemmbalken 1 wird auf diese Weise unterstützt. Die Rippen 10 unterstützen damit zugleich die in der Chirurgie gebrauchte Anwendung des ersten Klemmbalkens 1 als festen, kippsicheren Haken zur Darstellung des zu clippenden Gewebes bei offenem Clip, was mit einer herausragenden Effizienzsteigerung in der Genauigkeit und Sicherheit der Clippung verbunden ist.
Bei geschlossener Gewebeklammer wird durch den unter den korrespondierenden Klemmbalken greifenden Schenkel 5 die Biegebeanspruchung des Haltestiftes 4 wesentlich gemindert, wenn nicht sogar aufgehoben, verbunden mit einer signifikanten Steigerung der Stabilität der Gewebeklammer.
Für die Hakenfunktion ist von besonderer Bedeutung, dass die Biegebeanspruchung des Haltestiftes 4 wesentlich gemindert oder sogar aufgehoben ist.
Für die Anwendung eines derartig ausgebildeten Clips ist es vorteilhaft, wenn sich die lichte Weite zwischen den Führungsnuten 13, 22 (**Fig. 9**) zu ihrem distalen Ende verringert und/oder die Seitenteile der Kartusche 11 zu ihrem distalen Ende hin elastisch ausgebildet sind.

Der Grad der stufenlosen Klemmung des Gewebes kann durch den Anstellwinkel der Klemmflächen der Schultern 6, 7 und der korrespondierenden Nut 13, 22 der Kartusche 11 bei deren Fertigung variiert und dem jeweils gewünschten Anpressdruck auf die Klemmbalken 1, 2 angepasst werden. Des Weiteren ist der Grad der Pressklemmung durch die Auswahl eines Materials für die Klemmbalken 1, 2 und der Kartusche 11 mit geeignetem Elastizitätsmodul einstellbar.

Die **Fig. 7** zeigt in einem teilweisen Längsschnitt in einer prinzipiellen Darstellung einen am distalen Ende einer Kartusche 11 positionierten ersten Klemmbalken 1 mit seinen zum Teil abgeflachten Schultern. Das distale Ende der Kartusche 11 ist elastisch ausgebildet und weist eine zum distalen Ende hin abnehmende lichte Weite der Führungsnuten 13 auf. Die Kartusche 11 besitzt beidseitig einen über einen Teil oder die gesamte Breite der Schultern des ersten Klemmbalkens 1 reichenden Auswurfkanal. Im Unterschied dazu müssen bei bekannten technischen Lösungen die zur Führung von Klemmbalken angebrachten Führungsstifte zum Auswerfen der Gewebeklammer abgeschert werden.

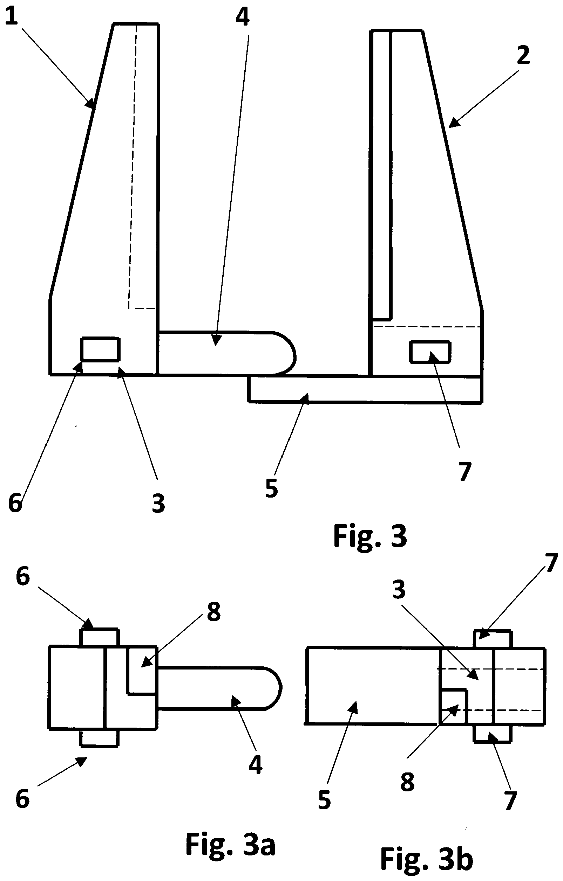

Aus den **Figuren 3, 3a** und **3b** geht eine weitere Ausführungsform der Erfindung hervor. Die Schultern 6, 7 erstrecken sich jeweils nicht über die gesamte Breite der Basis der Klemmbalken 1, 2. Die Klemmflächen 8 der Klemmbalken 1, 2 sind hier korrespondierend stufenförmig ausgebildet und greifen insofern beim Clippen des Gewebes ineinander. Über die gesamte Längsbreite der Klemmbalken 1, 2 sich erstreckende Schultern 6, 7 können auch geteilt sein.

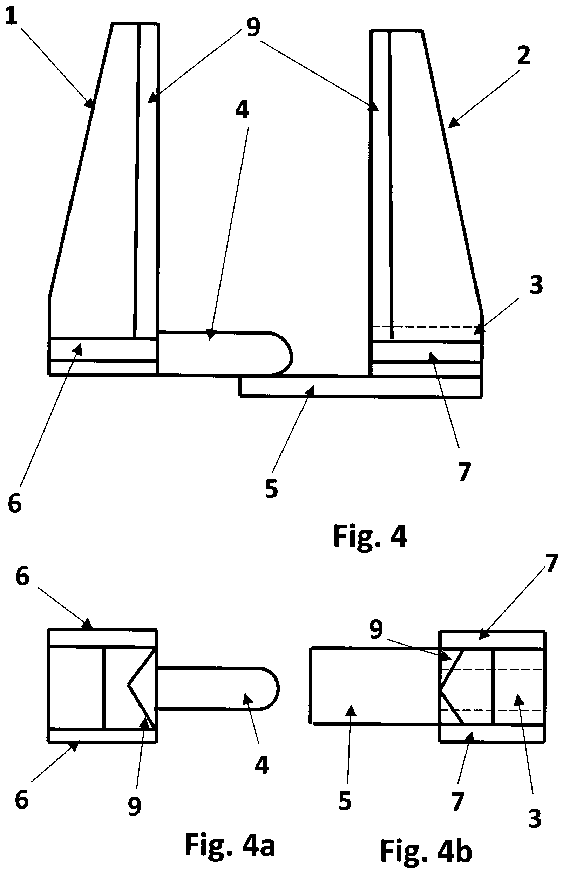

Die **Figuren 4, 4a** und **4b** zeigen ebenfalls eine Ausführungsform der Erfindung mit einer speziellen Formgebung der Klemmflächen 9 der Klemmbalken 1, 2. Die im Querschnitt eines stumpfen Winkels ausgebildete Klemmfläche 9 des zweiten Klemmbalkens 2 greift zum Clippen in eine korrespondierend ausgebildete Form des Querschnitts der Klemmfläche 9 des ersten Klemmbalkens 1. Eine derartige Ausbildung der Klemmflächen 9 unterstützt eine sichere Fixierung des Gewebes vor allem wenn noch hinzukommt, dass diese Flächen 9 eine aufgeraute Oberfläche aufweisen.

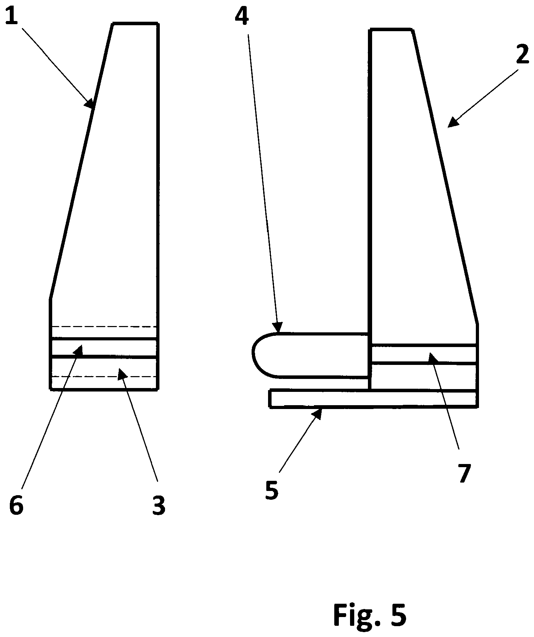

Wie aus **Fig. 5** zu entnehmen ist, kann der Haltestift 4 zusammen mit dem erfindungswesentlichen Schenkel 5, der vorzugsweise die gleiche Breite wie der zweite Klemmbalken 2 aufweist, auch an diesem vorgesehen sein, wie auch der erste Klemmbalken 1 sowohl den Haltestift 4, den Schenkel 5 und der dann kürzere Klemmbalken 2 die Haltebohrung 3 aufweisen kann.

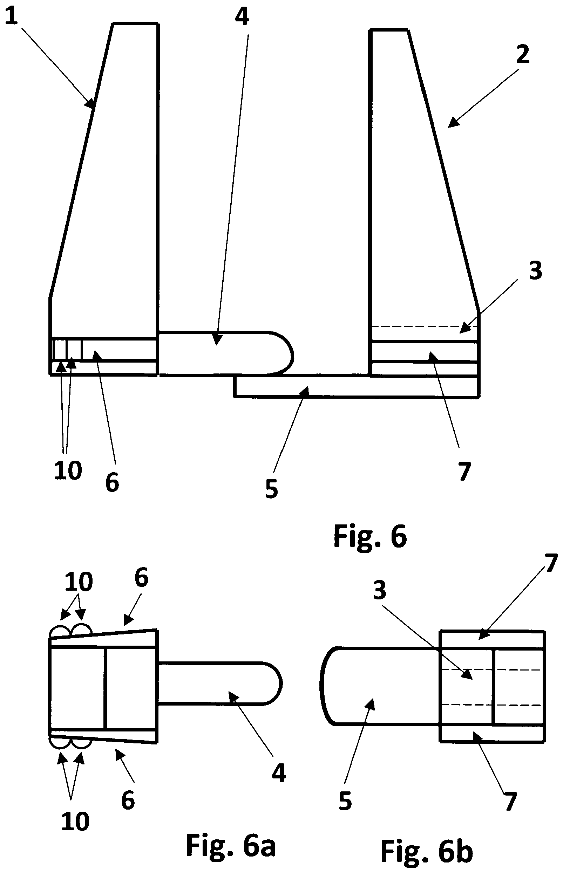

Die **Figuren 6, 6a** und **6b** stellen eine weitere Ausführungsform der Erfindung dar. Die Schultern 6 des ersten Klemmbalkens 1 weisen neben ihrer zum distalen Ende verlaufenden Abflachung zusätzlich eine vertikal verlaufende Rippung 10 bestehend aus einer Rippe oder mehreren Rippen auf. Die Seitenteile der Kartusche 11 besitzen zu der Rippung 10 jeweils passfähige Aufnahmen.

Es versteht sich, dass der erfindungsgemäße Clip die bei den **Figuren 1** bis **7** aufgezeigten technischen Merkmale in unterschiedlicher Kombination enthalten kann. So kann zum Beispiel die Rippung 10 auch an den nicht abgeflachten Schultern 6 und 7 der Klemmbalken 1, 2 vorgesehen sein. Der Schenkel 5 kann auch am ersten Klemmbalken 1 angeordnet sein. Beim Zusammenführen der Klemmbalken 1 und 2 schiebt sich in diesem Fall der dann kürzere zweite Klemmbalken 2 mit seinem unteren Ende bündig über den Schenkel 5 und klemmt dabei Weichteilgewebe oder Blutgefäße ab oder unterbricht diese.

Die **Fig. 8** zeigt in prinzipieller Darstellung eine zum Applizieren des erfindungsgemäßen Clips verwendeten Kartusche 11 als dafür geeignete Vorrichtung.

Die Klemmbalken 1, 2 wurden über die beidseitig vorhandenen Einsatzöffnungen 21 in die Kartuschen 11 eingeführt. Die Kartusche 11 kann aber auch getrennt voneinander jeweils Einsatzöffnungen für den ersten Klemmbalken 1 und für den zweiten Klemmbalken 2 aufweisen. Mittels des Stößels 14 ist der Klemmbalken 2 horizontal verschiebbar und kann die nach Zusammenfügen mit dem ersten Klemmbalken 1 unter Einschluss von organischem Gewebe gebildete Gewebeklammer ausgestoßen werden. An ihrem proximalen Ende ist die Kartusche 11 in Gestalt eines Hüllrohrabschnittes 17 ausgebildet. Durch den Hüllrohrabschnitt 17 wird die Kartusche 11 mit dem Stößel 14 bestückt. Andererseits dient der Hüllrohrabschnitt 17 der Kopplung mit dem hier nicht dargestellten Applikator als Handhabungsinstrument. Das für einen Bajonettverschluss ausgebildete rohrförmige Ende des Applikators wird dazu auf den Hüllrohrabschnitt 17 bis zum Anschlag 15 geschoben und mittels des Fixierelementes 16 stabil positioniert.

Mit **Fig. 9** ist im Prinzip die Vorderansicht der Kartusche 11 gemäß **Fig. 8** bestückt mit einem Clip gemäß **Fig. 1** dargestellt. Der erste Klemmbalken 1 ist in den Nuten 13 und 22 der Kartusche 11 positioniert und wird in diesen geführt. Der Schenkel 5 des zweiten Klemmbalkens 2 gleitet auf dem Bett 12 der Kartusche 11. Deutlich ist zu erkennen, dass die Nut 13 und die Nut 22 durchlaufend ausgeführt und zum distalen Ende der Kartusche 11 offen sind. Um die Pressklemmung des ersten Klemmbalkens 1 in der Kartusche 11 zu gewährleisten, verringert sich bei diesem Ausführungsbeispiel die durch die Nut 13 und die Nut 22 gebildete lichte Weite in Richtung des distalen Endes der Kartusche 11. Bei einer solchen Ausführung der Kartusche 11 gemäß **Fig. 7** können unterstützend für die Pressklemmung das distale Ende der Kartusche 11 und/oder die Schultern 6, 7 der Klemmbalken 1, 2 aus einem elastischen Material geformt sein. Die Pressklemmung des ersten Klemmbalkens 1 kann allein auch schon durch ausgeformte und vertikal verlaufende Rippen 10, die in passfähige Ausnehmungen in den Führungsnuten 13, 22 bzw. den Schultern 6, 7 greifen, erreicht werden.

Ein für die Kartusche 11 einsetzbarer Stößel 14 ist in Seitenansicht in **Fig. 10a** und mit deren Vorderansicht in **Fig. 10b** im Prinzip dargestellt. Der Stößel 14 weist beidseitig die Schultern 20 auf, die ebenfalls in den Nuten 13 und 22 gelagert und mittels des nicht dargestellten Applikators geführt werden können. Zur sicheren anfänglichen Positionierung des Stößels 14 im Hüllrohrabschnitt 17 kann der untere Steg 23 des Stößels 14 eine federnd ausgebildete Zunge 19 aufweisen, die mittels des Fixierelementes 18 in eine hier nicht dargestellte korrespondierende Ausnehmung des Hüllrohrabschnittes 17 einrastet. Um ein Ausstoßen des Stößels 14 aus der Kartusche 11 zusammen mit der Gewebeklammer zu vermeiden weist die Kartusche 11 an ihrem Boden zwischen dem distalen Ende und dem als ringsförmige Wulst ausgebildeten Anschlag 15 mindestens eine weitere ebenfalls hier nicht dargestellte Ausnehmung auf, in die das Fixierelement 18 nach dem Ausstoß der Gewebeklammer einrastet, so dass der Stößel 14 nicht ausgeworfen werden kann.

### BEZUGSZEICHENAUFSTELLUNG

- 1: Klemmbalken;
- 2: Klemmbalken;
- 3: Haltebohrung;
- 4: Haltestift;
- 5: Schenkel;
- 6: Schulter;
- 7: Schulter;
- 8: Aussparung;
- 9: Klemmfläche;
- 10: Rippen
- 11: Kartusche
- 12: Kartuschenbett
- 13: Nut
- 14: Stößel
- 15: Anschlag
- 16: Fixierelement
- 17: Hüllrohrabschnitt
- 18: Fixierelement
- 19: Federzunge
- 20: Schulter
- 21: Einsatzöffnung
- 22: Nut
- 23: Steg

## Patentansprüche

1. Anordnung mit einem chirurgischen Clip für die minimal invasive Chirurgie zum Abklemmen von organischem Gewebe zu einer Gewebeklammer und mit einer Kartusche (11) zum Applizieren des chirurgischen Clips, wobei der chirurgische Clip aus einem ersten Klemmbalken (1) korrespondierend mit einem zweiten Klemmbalken (2) besteht, wobei der erste Klemmbalken (1) und der zweite Klemmbalken (2) an ihrer Basis beidseitig jeweils Schultern (6, 7) für deren Führung in Nuten (13) der Kartusche (11) aufweisen und wobei ein Klemmbalken (2) eine durchgehende Haltebohrung (3) aufweist, in die zum Abklemmen des organischen Gewebes kraft- und formschlüssig ein am anderen Klemmbalken (1) vorgesehener Haltestift (4) greift, wobei
- die Kartusche (11) mindestens eine Ausnehmung (21) zur Aufnahme der Klemmbalken (1, 2) aufweist,
- der zweite Klemmbalken (2) in einer Ebene mit dem ersten Klemmbalken (1) unter Pressklemmung des Gewebes stufenlos mit dem ersten Klemmbalken (1) zusammenfügbar ist,
- in der Kartusche (11) ein Stößel (14) zum Verschieben des zweiten Klemmbalkens (2) und zum Ausstoßen der Gewebeklammer gelagert ist und die Kartusche (11) an ihrem proximalen Ende Mittel für eine lösbare Verbindung mit einem Applikator aufweist;
- der erste Klemmbalken (1) und der zweite Klemmbalken (2) jeweils mit den Schultern (6; 7) aus einem resorbierbarem Material bestehen,
**dadurch gekennzeichnet, dass**
- der erste Klemmbalken (1) mittels Pressklemmung am distalen Ende der Kartusche (11) positioniert ist;
- der erste Klemmbalken (1) oder der zweite Klemmbalken (2) an seinem unteren Ende einen Schenkel (5) besitzt, der sich beim Zusammenfügen mit dem jeweils anderen Klemmbalken (1; 2) unter Abklemmen des organischen Gewebes formschlüssig unter den kürzeren anderen Klemmbalken (1; 2) schiebt und so der Gewebeklammer eine erhebliche zusätzliche Stabilität gibt; und
- die Nuten (13; 22) zum distalen Ende der Kartusche (11) offen ausgebildet sind.

2. Anordnung nach Anspruch 1, **dadurch gekennzeichnet, dass** die Schultern des ersten Klemmbalkens (1) zum distalen Ende hin über ihre gesamte Länge oder nur teilweise abgeflacht ausgebildet sind, jeweils korrespondierend mit einer Verringerung der lichten Weite zwischen den Nuten (13; 22) der Kartusche (11).

3. Anordnung nach Anspruch 1 oder 2, **dadurch gekennzeichnet, dass** die Klemmflache (8) des ersten Klemmbalkens (1) und die des zweiten Klemmbalkens (2; 14) aufgeraut oder wellenförmig ausgebildet sind.

4. Anordnung nach einem der Anspruche 1 bis 3, **dadurch gekennzeichnet, dass** die Klemmfläche (8) des ersten Klemmbalkens (1) und die Klemmfläche (8) des korrespondierenden zweiten Klemmbalkens (2) stufenförmig ausgebildet sind.

5. Anordnung nach einem der Ansprüche 1 bis 4, **dadurch gekennzeichnet, dass** der Schenkel (5) eine Länge aufweist, die mindestens der Längenbreite des korrespondierenden ersten Klemmbalkens (1) an dessen Basis entspricht.

6. Anordnung nach einem der Ansprüche 1 bis 5, **dadurch gekennzeichnet, dass** die Schultern (6) des ersten Klemmbalkens (1) vertikal verlaufende Rippungen (10) aufweisen.

7. Anordnung nach einem der Ansprüche 1 bis 6, **dadurch gekennzeichnet, dass** die Klemmpressung des ersten Klemmbalkens (1) durch die in Richtung des distalen Endes der Kartusche (11) verlaufende Verringerung der lichten Weite zwischen den Führungsnuten (13; 22) und/oder durch die Rippen (10) der Schultern (6) des ersten Klemmbalkens (1) bewirkt ist, die in die Ausnehmungen greifen, die in den Führungsnuten (13; 22) am distalen Ende der Kartusche (11) dafür vorgesehen sind.

8. Anordnung nach einem der Ansprüche 1 bis 7, **dadurch gekennzeichnet, dass** die Breite des Schenkels (5) der Breite eines Bettes (12) der Kartusche (11) entspricht.

9. Anordnung nach einem der Ansprüche 1 bis 8, **dadurch gekennzeichnet, dass** die Schultern (6; 7) des ersten Klemmbalkens (1) und des zweiten Klemmbalkens (2) eine solche Elastizität aufweisen, dass die Gewebeklammer auch bei starren distalen Enden der Kartusche (11) durch Pressdruck unbeschadet auswerfbar ist.

10. Anordnung nach einem der Ansprüche 1 bis 9, **dadurch gekennzeichnet, dass** die Schultern (6; 7) des ersten Klemmbalkens (1) und des zweiten Klemmbalkens (2) aus einem unelastischem Material bestehen und das distale Ende der Kartusche (11) elastisch ausgebildet ist und so einen unbeschadeten Auswurf der Gewebeklammer ermöglicht.

11. Anordnung nach Anspruch 1, **dadurch gekennzeichnet, dass** das Mittel zur lösbaren Verbindung mit dem Applikator ein Anschlag (15) und ein am unteren Ende eines Hüllrohrabschnittes (17) der Kartusche (1) vorgesehenes Fixierelement (16) für die Aufnahme des Applikators mittels eines Bajonettverschlusses oder das Mittel Rastelemente sind.

12. Anordnung nach einem der Ansprüche 1 oder 11, **dadurch gekennzeichnet, dass** der Stößel (14) ein Fixierelement (18) aufweist, das bei Handhabung des chirurgischen Clips in korrespondierende Ausnehmungen greift, die mit Abstand voneinander in Längsrichtung im Boden des Hüllrohrabschnittes (17) vorgesehen sind.

## Claims

1. Assembly having a surgical clip for minimally invasive surgery, for clamping off organic tissue and forming a tissue clamp, and having a cartridge (11) for applying the surgical clip, wherein the surgical clip consists of a first clamping bar (1) corresponding to a second clamping bar (2), wherein the first clamping bar (1) and the second clamping bar (2) have at their base, on both sides in each case, shoulders (6, 7) for guiding them in grooves (13) of the cartridge (11), and wherein one clamping bar (2) has a continuous holding bore (3) in which a holding pin (4) provided on the other clamping bar (1) engages with a force fit and form fit in order to clamp off the organic tissue, wherein
- the cartridge (11) has at least one recess (21) for receiving the clamping bars (1, 2),
- the second clamping bar (2), in a plane with the first clamping bar (1), can be joined together steplessly with the first clamping bar (1) to press clamp the tissue;
- a plunger (14) for displacing the second clamping bar (2) and for ejecting the tissue clamp is mounted in the cartridge (11), and the cartridge (11) has, at its proximal end, means for a releasable connection to an applicator;
- the first clamping bar (1) and the second clamping bar (2), in each case with the shoulders (6; 7), are made of a resorbable material,
**characterized in that**
- the first clamping bar (1) is positioned at the distal end of the cartridge (11) by means of press clamping;
- the first clamping bar (1) or the second clamping bar (2) has, at its lower end, a limb (5) which, during the joining together with the respective other clamping bar (1; 2), slides with form-fit engagement under the shorter, other clamping bar (1; 2), to clamp off the organic tissue, and in so doing gives the tissue clamp a considerable additional stability;
and
- the grooves (13; 22) are open to the distal end of the cartridge (11).

2. Assembly according to Claim 1, **characterized in that** the shoulders of the first clamping bar (1) are configured flattened towards the distal end, over their entire length or only in part, corresponding in each case to a reduction in the clear width between the grooves (13; 22) of the cartridge (11).

3. Assembly according to Claim 1 or 2, **characterized in that** the clamping face (8) of the first clamping bar (1) and that of the second clamping bar (2; 14) have a roughened or corrugated configuration.

4. Assembly according to one of Claims 1 to 3, **characterized in that** the clamping face (8) of the first clamping bar (1) and the clamping face (8) of the corresponding second clamping bar (2) have a step-shaped configuration.

5. Assembly according to one of Claims 1 to 4, **characterized in that** the limb (5) has a length which corresponds at least to the length width of the corresponding first clamping bar (1) at the base thereof.

6. Assembly according to one of Claims 1 to 5, **characterized in that** the shoulders (6) of the first clamping bar (1) have vertically extending ribs (10).

7. Assembly according to one of Claims 1 to 6, **characterized in that** the press clamping of the first clamping bar (1) is effected by the reduction in the clear width between the guide grooves (13; 22) in the direction of the distal end of the cartridge (11) and/or by the ribs (10) of the shoulders (6) of the first clamping bar (1) that engage in the recesses which are provided for this purpose in the guide grooves (13; 22) at the distal end of the cartridge (11).

8. Assembly according to one of Claims 1 to 7, **characterized in that** the width of the limb (5) corresponds to the width of a bed (12) of the cartridge (11).

9. Assembly according to one of Claims 1 to 8, **characterized in that** the shoulders (6; 7) of the first clamping bar (1) and of the second clamping bar (2) have such elasticity that, even if the distal ends of the cartridge (11) are rigid, the tissue clamp can be ejected by a pressing force without sustaining damage.

10. Assembly according to one of Claims 1 to 9, **characterized in that** the shoulders (6; 7) of the first clamping bar (1) and of the second clamping bar (2) are made of a non-elastic material, and the distal end of the cartridge (11) is configured to be elastic so as to permit ejection of the tissue clamp without sustaining damage.

11. Assembly according to Claim 1, **characterized in that** the means for the releasable connection to the applicator are a stop (15) and a fixing element (16) provided at the lower end of a sheathing tube portion (17) of the cartridge (1) for receiving the applicator by means of a bayonet catch or the means are latching elements.

12. Assembly according to either of Claims 1 and 11, **characterized in that** the plunger (14) has a fixing element (18) which, during the handling of the surgical clip, engages in corresponding recesses which are provided, at a distance from one another in the longitudinal direction, in the bottom of the sheathing tube portion (17).

## Revendications

1. Agencement avec une pince chirurgicale pour chirurgie mini-invasive, destiné à clamper des tissus organiques en pince pour tissus, et avec une cartouche (11) pour appliquer la pince chirurgicale, dans lequel la pince chirurgicale est composée d'une première barrette de serrage (1) correspondant à une deuxième barrette de serrage (2), dans lequel la première barrette de serrage (1) et la deuxième barrette de serrage (2) présentent à leur base des deux côtés respectivement des épaulements (6, 7) destinés à les guider dans des rainures (13) de la cartouche (11), et dans lequel une barrette de serrage (2) présente un perçage de maintien traversant (3) dans lequel, pour clamper les tissus organiques, une goupille de maintien (4) prévue sur l'autre barrette de serrage (1) s'engage par adhérence et par complémentarité de forme, dans lequel
- la cartouche (11) présente au moins un évidement (21) pour recevoir la barrette de serrage (1, 2),
- la deuxième barrette de serrage (2), dans un plan avec la première barrette de serrage (1), peut être assemblée avec la première barrette de serrage (1) en continu par serrage par pression des tissus,
- un poussoir (14) pour déplacer la deuxième barrette de serrage (2) et pour éjecter la pince pour tissus est logé dans la cartouche (11), et la cartouche (11) présente à son extrémité proximale des moyens pour une connexion amovible avec un applicateur ;
- la première barrette de serrage (1) et la deuxième barrette de serrage (2), respectivement avec les épaulements (6 ; 7), sont composées d'un matériau résorbable,
**caractérisé en ce que**
- la première barrette de serrage (1) est positionnée au moyen d'un serrage par pression à l'extrémité distale de la cartouche (11) ;
- la première barrette de serrage (1) ou la deuxième barrette de serrage (2) dispose à son extrémité inférieure d'une branche (5) qui, lors de l'assemblage avec l'autre barrette de serrage respective (1 ; 2), passe par complémentarité de forme sous l'autre barrette de serrage (1 ; 2) plus courte en clampant les tissus organiques, conférant ainsi une stabilité supplémentaire considérable à la pince pour tissus ; et
- les rainures (13 ; 22) sont réalisées de manière à être ouvertes vers l'extrémité distale de la cartouche (11).

2. Agencement selon la revendication 1, **caractérisé en ce que** les épaulements de la première barrette de serrage (1) vers l'extrémité distale sont réalisés de manière aplatie sur toute sa longueur ou seulement en partie, respectivement en correspondance avec une diminution de la largeur libre entre les rainures (13 ; 22) de la cartouche (11).

3. Agencement selon la revendication 1 ou 2, **caractérisé en ce que** la surface de serrage (8) de la première barrette de serrage (1) et celle de la deuxième barrette de serrage (2 ; 14) sont réalisées de manière striée ou ondulée.

4. Agencement selon l'une quelconque des revendications 1 à 3, **caractérisé en ce que** la surface de serrage (8) de la première barrette de serrage (1) et la surface de serrage (8) de la deuxième barrette de serrage (2) correspondante sont réalisées en échelons.

5. Agencement selon l'une quelconque des revendications 1 à 4, **caractérisé en ce que** la branche (5) présente une longueur correspondant au moins à la largeur longitudinale de la première barrette de serrage (1) correspondante à la base de celle-ci.

6. Agencement selon l'une quelconque des revendications 1 à 5, **caractérisé en ce que** les épaulements (6) de la première barrette de serrage (1) présentent des nervures (10) s'étendant verticalement.

7. Agencement selon l'une quelconque des revendications 1 à 6, **caractérisé en ce que** le serrage par pression de la première barrette de serrage (1) est provoqué par la diminution de la largeur libre entre les rainures de guidage (13 ; 22) s'étendant vers l'extrémité distale de la cartouche (11) et/ou par les nervures (10) des épaulements (6) de la première barrette de serrage (1) qui s'engagent dans les évidements prévus à cet effet dans les rainures de guidage (13 ; 22) à l'extrémité distale de la cartouche (11).

8. Agencement selon l'une quelconque des revendications 1 à 7, **caractérisé en ce que** la largeur de la branche (5) correspond à la largeur d'un lit (12) de la cartouche (11).

9. Agencement selon l'une quelconque des revendications 1 à 8, **caractérisé en ce que** les épaulements (6 ; 7) de la première barrette de serrage (1) et de la deuxième barrette de serrage (2) présentent une élasticité telle que la pince pour tissus peut être éjectée sans dommage par pression de pressage même en cas d'extrémités distales rigides de la cartouche (11).

10. Agencement selon l'une quelconque des revendications 1 à 9, **caractérisé en ce que** les épaulements (6 ; 7) de la première barrette de serrage (1) et de la deuxième barrette de serrage (2) sont composés d'un matériau non élastique et l'extrémité distale de la cartouche (11) est réalisée de manière élastique et permet donc une éjection sans dommage de la pince pour tissus.

11. Agencement selon la revendication 1, **caractérisé en ce que** les moyens pour la connexion amovible avec l'applicateur correspondent à une butée (15) et à un élément de fixation (16) prévu à l'extrémité inférieure d'une partie de tube de gainage (17) de la cartouche (1) pour recevoir l'applicateur au moyen d'un joint à baïonnette ou **en ce que** les moyens sont des éléments d'arrêt.

12. Agencement selon l'une quelconque des revendications 1 ou 11, **caractérisé en ce que** le poussoir (14) présente un élément de fixation (18) qui, lors de la manipulation de la pince chirurgicale, s'engage dans des évidements correspondants prévus à distance les uns des autres dans la direction longitudinale dans le fond de la partie de tube de gainage (17).
